# EUROPEAN PATENT APPLICATION

(11) **EP 4 249 522 A1**
(43) Date of publication of application: **27.09.2023**
(21) Application number: 21915103.2
(22) Date of filing: 15.12.2021
(51) Int. Cl.: C08F 8/12, A61F 13/53, A61L 15/26

(54) **COMPOSITE ABSORBENT OBJECT AND SANITARY SUPPLY**

(30) Priority: 29.12.2020 JP 2020219815
(71) Applicant: Unicharm Corporation, Shikokuchuo-shi, Ehime 799-0111 (JP)
(72) Inventor: KIKUCHI, Kyo, Kanonji-shi, Kagawa 769-1602 (JP); KINOSHITA, Akie, Kanonji-shi, Kagawa 769-1602 (JP); NAKASHITA, Masashi, Kanonji-shi, Kagawa 769-1602 (JP); GODA, Hiroki, Kanonji-shi, Kagawa 769-1602 (JP); UKEGAWA, Kazuo, Kanonji-shi, Kagawa 769-1602 (JP); KUNITOMO, Teruo, Kanonji-shi, Kagawa 769-1602 (JP)
(74) Representative: Dolleymores
(86) International application number: PCT/JP2021/046333
(87) International publication number: WO 2022/145242

(57) **Abstract**

The present invention provides an absorbent object capable of stably exhibiting absorption performance. This composite absorbent object (4) is for absorbing bodily fluids and is characterized by comprising a superabsorbent polymer and a polymeric absorber including a hydrophilic continuous skeleton and having interconnected voids the polymeric absorber at least containing -COOH and -COONa groups as ion-exchanging groups and having a total ion exchange capacity of the -COOH and -COONa groups of 4.0 mg-eq/g or greater per unit dried-state mass.

## Description

### FIELD

The present invention relates to a composite absorbent body and a sanitary product having the same.

### BACKGROUND

In sanitary products such as disposable diapers and sanitary napkins, those using a superabsorbent polymer (so-called "SAP") having a high absorption amount are known. For example, Patent Literature 1 discloses an absorbent article using an absorbent body formed by combining absorbent resin particles (superabsorbent polymer) having an excellent absorption amount and hydrophilic fibers such as pulp fibers having an excellent absorption rate.

### [CITATION LIST]

### [PATENT LITERATURE]

Patent Literature 1: PTC International Publication No. WO 2013/018571

### SUMMARY

### [TECHNICAL PROBLEM]

While such a superabsorbent polymer (SAP) can retain a large amount of water (that is, having a high water retention capacity), the superabsorbent polymer has a slow water absorption rate and is thus used in conjunction with the pulp to temporarily and rapidly retain water in conventional absorbent bodies. In the conventional absorbent bodies, when a bodily fluid such as urine is discharged from a wearer of the sanitary product, the bodily fluid is rapidly absorbed by the pulp in the absorbent body, temporarily held in the pulp, then transferred to SAP having a high water retention capacity, and held in the SAP.

On the other hand, in the bodily fluid, such as urine, which is an object to be absorbed by the absorbent body, the salt concentration in the bodily fluid varies greatly depending on differences in dietary habits (for example, ingredients of taken food and drink and the like) and living environments (for example, frequency of sweating and urination and the like), and in particular, divalent ions (for example, Ca²⁺, Mg²⁺, and the like) in the bodily fluid, even in a very small amount, have a large adverse effect on the absorption performance (particularly, water absorption amount, water retention amount, and absorption rate) of the SAP.

However, since the conventional absorbent body does not have a function of modifying the salt concentration in the bodily fluid such as urine, the bodily fluid temporarily held in the pulp is transferred to the SAP as it is to cause variation in the absorption performance of the SAP due to the influence of the salt concentration in the bodily fluid (particularly, divalent ion concentration), resulting in a risk that the absorption performance of the absorbent body cannot be stably exhibited.

The present invention was achieved in view of such problems, and an aspect of the present invention is to provide an absorbent body capable of stably exhibiting absorption performance.

### [SOLUTION TO PROBLEM]

One aspect (Aspect 1) of the present invention is:
A composite absorbent body for absorbing a bodily fluid, the composite absorbent body comprising:
a polymer absorbent having a hydrophilic continuous skeleton and continuous pores; and
a superabsorbent polymer, wherein
the polymer absorbent contains at least a -COOH group and a -COONa group as ion exchange groups, and
a total ion exchange capacity of the -COOH group and the -COONa group per unit mass in a dry state is 4.0 mg equivalent/g or more.

Since the polymer absorbent has a hydrophilic continuous skeleton and continuous pores, the composite absorbent body according to the present aspect can rapidly absorb and temporarily hold a bodily fluid such as urine. Further, since the polymer absorbent contains a certain amount or more of -COOH groups and -COONa groups, which are ion exchange groups, when the polymer absorbent absorbs and temporarily holds the bodily fluid, the ions (particularly, divalent ions such as Ca²⁺ and Mg²⁺) in the bodily fluid can be ion-exchanged with the -COOH group and the - COONa group to modify the bodily fluid into a bodily fluid that is less likely to have an adverse effect on the absorption performance (particularly, water absorption amount, water retention amount, and absorption rate) of the superabsorbent polymer (SAP).

Accordingly, since the composite absorbent body according to the present aspect transfers the bodily fluid to the SAP after the bodily fluid is modified with the polymer absorbent, variation in the absorption performance of the SAP is less likely to occur, and the absorption performance can be stably exhibited as an absorbent body.

In addition, another aspect (Aspect 2) of the present invention is:
the composite absorbent body according to Aspect 1, wherein
an ion exchange rate of polyvalent ions of the polymer absorbent is 50% or more.

In the composite absorbent body according to the present aspect, since the ion exchange rate of the polyvalent ions (ions having a valence of 2 or more) of the polymer absorbent is 50% or more and the bodily fluid can be more reliably modified, variation in the absorption performance of the SAP can be made much less likely to occur, and the absorption performance can be more stably exhibited as an absorbent body.

Still another aspect (Aspect 3) of the present invention is:
the composite absorbent body according to Aspect 1 or 2, wherein
a water absorption amount per unit mass of the polymer absorbent is 30 g/g or more.

In the composite absorbent body according to the present aspect, since the polymer absorbent has a certain water absorption amount or more and can absorb and steadily modify a larger amount of bodily fluid, variation in the absorption performance of the SAP can be made much less likely to occur, and the absorption performance can be more stably exhibited as an absorbent body.

Still another aspect (Aspect 4) of the present invention is:
the composite absorbent body according to any one of Aspects 1 to 3, wherein
in the polymer absorbent, a porosity per unit volume of the polymer absorbent is 85% or more.

In the composite absorbent body according to the present aspect, since the polymer absorbent has a certain porosity or higher and can absorb and steadily modify a larger amount of bodily fluid, variation in the absorption performance of the SAP can be made much less likely to occur, and the absorption performance can be more stably and satisfactorily exhibited as an absorbent body.

Still another aspect (Aspect 5) of the present invention is:
the composite absorbent body according to any one of Aspects 1 to 4, wherein
an average diameter of the continuous pores of the polymer absorbent is 1 µm to 1000 µm.

In the composite absorbent body according to the present aspect, since the average diameter of the continuous pores of the polymer absorbent is within the above specific range, the spaces (pores) for taking in the bodily fluid of the polymer absorbent are less likely to collapse, and the composite absorbent body can have a higher absorption rate and stably exhibit excellent absorption performance.

Particularly, in a case where the porosity per unit volume of the polymer absorbent is 85% or more and the average diameter of the continuous pores is 1 µm to 1000 µm, the bodily fluid can be absorbed with a larger number of pores and modified, and thus there is an advantage that further excellent ion exchange efficiency can be embodied.

Still another aspect (Aspect 6) of the present invention is:
the composite absorbent body according to any one of Aspects 1 to 5, wherein
the polymer absorbent is a monolithic absorbent.

In the composite absorbent body according to the present aspect, since the polymer absorbent is a monolithic absorbent, the bodily fluid can be rapidly absorbed, and the temporarily held liquid can be more steadily transferred to the SAP, further excellent absorption performance can be stably exhibited.

Still another aspect (Aspect 7) of the present invention is:
the composite absorbent body according to any one of Aspects 1 to 6, wherein
the polymer absorbent is a hydrolysate of a crosslinked polymer of a (meth)acrylic acid ester and a compound containing two or more vinyl groups in one molecule.

In the composite absorbent body according to the present aspect, since the polymer absorbent has the above-described specific configuration, the hydrophilic continuous skeleton easily extends and the continuous pores are also easily widened when the polymer absorbent absorbs the bodily fluid. Therefore, a larger amount of bodily fluid can be more rapidly taken into the continuous pores, and the composite absorbent body can more reliably exhibit higher absorption performance as an absorbent body. Further, a larger amount of bodily fluid can be steadily modified, and variation in the absorption performance of the SAP can be made much less likely to occur.

Still another aspect (Aspect 8) of the present invention is:
the composite absorbent body according to any one of Aspects 1 to 7, wherein
the superabsorbent polymer is an acrylic acid-based superabsorbent polymer having cations present on a surface.

The absorption performance (particularly, the water absorption amount, water retention amount, and absorption rate) of the acrylic acid-based superabsorbent polymer (SAP) having cations present on the surface is particularly easily adversely affected by the ions in the bodily fluid. However, even in a case where the composite absorbent body according to the present aspect contains such SAP, when the polymer absorbent absorbs and temporarily holds the bodily fluid, the ions in the bodily fluid can be ion-exchanged with the -COOH group and the -COONa group to modify the bodily fluid. Therefore, variation in the absorption performance of the SAP is less likely to occur, and the absorption performance can be stably exhibited as an absorbent body.

Still another (Aspect 9) of the present invention is:
a sanitary product comprising:
the composite absorbent body according to any one of Aspects 1 to 8.

Since the sanitary product of the present aspect has the composite absorbent body according to any of Aspects 1 to 8 described above, high absorption performance (for example, absorption performance such as difficulty in occurrence of rewetting, and high absorption rate) can be exhibited as a sanitary product.

### [ADVANTAGEOUS EFFECT OF THE INVENTION]

The present invention can provide an absorbent body capable of stably exhibiting absorption performance.

### BRIEF DESCRIPTION OF DRAWINGS

FIG. 1 is a schematic plan view of a light incontinence pad 1 in an unfolded state when viewed in the thickness direction from the skin facing surface side.
FIG. 2 is a diagram illustrating a manufacturing process of an absorbent A which is an example of a polymer absorbent.
FIG. 3 is an SEM photograph of the absorbent A at a magnification of 50 times.
FIG. 4 is an SEM photograph of the absorbent A at a magnification of 100 times.
FIG. 5 is an SEM photograph of the absorbent A at a magnification of 500 times.
FIG. 6 is an SEM photograph of the absorbent A at a magnification of 1000 times.
FIG. 7 is an SEM photograph of the absorbent A at a magnification of 1500 times.
FIG. 8 is a graph showing the relationship between the monovalent and divalent ion concentration in a bodily fluid (urine) and the absorption performance (water absorption amount, water retention amount, and absorption rate) of SAP.
FIG. 9 is a graph showing the influence of the absorbent A, which is an example of a polymer absorbent, on the divalent ion concentration in the bodily fluid (urine).

### DESCRIPTION OF EMBODIMENTS

Hereinafter, a preferable embodiment of a composite absorbent body of the present invention will be described in detail using a light incontinence pad 1 which is an example of a sanitary product to which the composite absorbent body is applied.

It should be noted that in the present specification, unless otherwise specified, an "object (for example, a light incontinence pad, a composite absorbent body, or the like) placed on a horizontal plane in an unfolded state is viewed in a thickness direction of the object from the upper side in the vertical direction (in a case where the object is a sanitary product, the top sheet side)" will be simply referred to as a "plan view".

It should be noted that in the present specification, the "lengthwise direction" refers to a "direction in which the length of a longitudinally elongated object (for example, a light incontinence pad, a composite absorbent body, or the like in an unfolded state) in a plan view is long", the "width direction" refers to a "direction in which the length of a longitudinally elongated object in a plan view is short", the "thickness direction" refers to a "direction vertical to the object placed on a horizontal plane in an unfolded state", and the lengthwise direction, the width direction, and the thickness direction have a relationship in which the respective directions are orthogonal to each other.

Further, in the present specification, unless otherwise specified, in the thickness direction of the light incontinence pad 1, a "relatively proximal side with respect to the skin surface of the wearer while the light incontinence pad 1 is put on" will be referred to as a "skin facing surface side," and a "relatively distal side with respect to the skin surface of the wearer while the light incontinence pad 1 is put on" will be referred to as a "non-skin facing surface side."

### [Light Incontinence Pad]

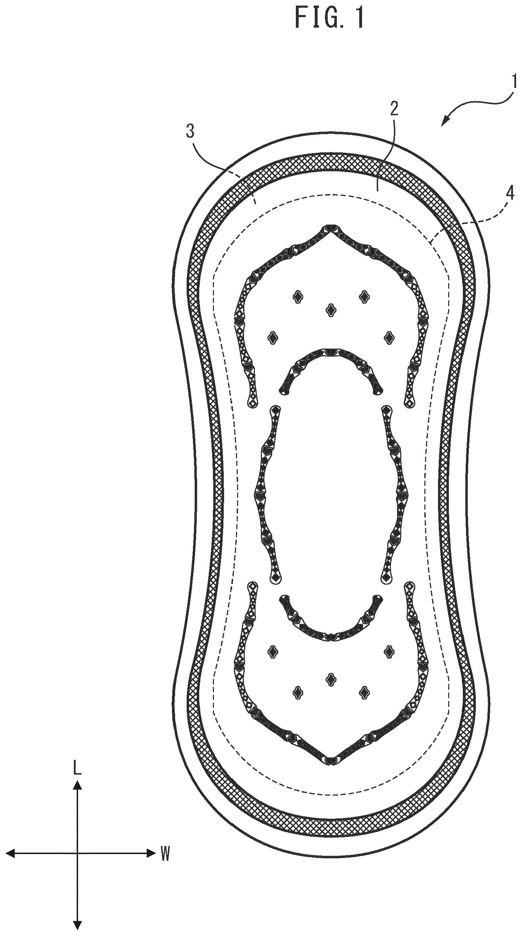

FIG. 1 is a schematic plan view of a light incontinence pad 1 in an unfolded state to which a composite absorbent body 4 according to an embodiment of the present invention is applied.

As shown in FIG. 1, in a plan view, the light incontinence pad 1 has a lengthwise direction L and a width direction W, and has a longitudinally elongated outer shape in which two lengthwise end edges protrude toward the outer side in the lengthwise direction in an arc shape. It should be noted that the outer shape of the light incontinence pad 1 is not limited to such an embodiment, and, as long as the light incontinence pad 1 has a longitudinally elongated shape, any shape (for example, an elliptical shape, a rectangular shape, a hourglass shape, a gourd shape, and the like) can be employed according to various uses, usage modes, and the like.

The light incontinence pad 1 basically includes, in a thickness direction, a liquid-permeable top sheet 2 that forms a surface on a skin facing surface side of the light incontinence pad 1, a back sheet 3 that forms a surface on a non-skin facing surface side of the light incontinence pad 1, and a composite absorbent body 4 that is positioned between the top sheet and the back sheet. Further, the light incontinence pad 1 further includes an adhesive portion (not shown) that is arranged on the surface of the non-skin facing surface side of the back sheet 3 and adhesively fixes the light incontinence pad 1 to the inner surface of clothing such underwear of a wearer.

It should be noted that the configuration of the light incontinence pad 1 is not limited to the above-described configuration, and, for example, the light incontinence pad 1 may include a pair of side sheets for forming leakproof walls, which are positioned at both end portions of the light incontinence pad 1 in the width direction W and arranged to extend in the lengthwise direction L, at a position on the skin facing surface side with respect to the top sheet 2, and a plurality of elastic members which are arranged along the lengthwise direction L in each of the pair of side sheets.

Further, in the light incontinence pad 1, the composite absorbent body 4 is formed of a water-absorbent member that is positioned between the top sheet 2 and the back sheet 3 and is capable of absorbing a bodily fluid such as urine discharged from the wearer and permeating the top sheet 2, and the composite absorbent body 4 includes a polymer absorbent having a hydrophilic continuous skeleton and continuous pores, and a superabsorbent polymer (SAP).

Further, the above-described polymer absorbent contains at least a -COOH group and a -COONa group as ion exchange groups, and has a unique ion exchange ability that the total ion exchange capacity of the -COOH group and the -COONa group per unit mass in a dry state is 4.0 mg equivalent/g or more.

Since the polymer absorbent 4 has a hydrophilic continuous skeleton and continuous pores, the composite absorbent body according to the present aspect can rapidly absorb and temporarily hold a bodily fluid such as urine. Further, since the polymer absorbent contains a certain amount or more of -COOH groups and -COONa groups, which are ion exchange groups, when the polymer absorbent absorbs and temporarily holds the bodily fluid, the ions (particularly, divalent ions such as Ca²⁺ and Mg²⁺) in the bodily fluid can be ion-exchanged with the -COOH group and the - COONa group to modify the bodily fluid into a bodily fluid that is less likely to have an adverse effect on the absorption performance (particularly, water absorption amount, water retention amount, and absorption rate) of the SAP.

Accordingly, since the composite absorbent body 4 transfers the bodily fluid to the SAP after modifying the bodily fluid with the polymer absorbent, variation in the absorption performance of the SAP is less likely to occur, and the absorption performance can be stably exhibited as an absorbent body.

Therefore, the light incontinence pad 1 including such a composite absorbent body 4 can also exhibit high absorption performance (for example, absorption performance such difficulty in occurrence of rewetting, and high absorption rate) as a light incontinence pad.

Hereinafter, various configurating members of a sanitary product to which the composite absorbent body of the present invention is applied will be described in more detail using the above-described light incontinence pad 1.

### [Top sheet]

In the above-described light incontinence pad 1, the top sheet 2 has a longitudinally elongated outer shape that extends from one side end edge to the other side end edge in the lengthwise direction L of the light incontinence pad 1, and extends over the vicinity of the one side end edge to the vicinity of the other side end edge in the width direction W of the light incontinence pad 1 in a plan view, as shown in FIG. 1. The top sheet 2 is formed of a liquid-permeable sheet-like member arranged at a position on the skin facing surface side in the thickness direction of the light incontinence pad 1 and forms a contact surface capable of coming into contact with the skin of the wearer, that is, the surface on the skin facing surface side of the light incontinence pad 1.

Further, as shown in FIG. 1, the top sheet 2 has a slightly larger size in the lengthwise direction L and the width direction W compared with the composite absorbent body 4 arranged on the non-skin facing surface side of the top sheet 2, and is joined to the back sheet 3 positioned on the non-skin facing surface side in the peripheral edge portion.

In the present invention, the outer shape, various dimensions, basis weight, and the like of the top sheet are not particularly limited, as long as they can be used for the top sheet of a sanitary product, and any shape, various dimensions, basis weight, and the like according to the desired liquid permeability, texture, flexibility, strength, and the like can be employed.

### [Back sheet]

In the above-described light incontinence pad 1, the back sheet 3 has a longitudinally elongated outer shape that extends from one side end edge to the other side end edge in the lengthwise direction L of the light incontinence pad 1 and extends from the one side end edge to the other side end edge in the width direction W of the light incontinence pad 1 in a plan view. The back sheet 3 is arranged at a position on the non-skin facing surface side in the thickness direction of the light incontinence pad 1, and is formed of a liquid-impermeable sheet-like member that forms the non-skin facing surface of the light incontinence pad 1 and prevents a bodily fluid such as urine that has permeated the composite absorbent body 4 from leaking out from the light incontinence pad 1.

In the present invention, the outer shape, various dimensions, basis weight, and the like of the back sheet are not particularly limited, as long as they can be used for the back sheet of the sanitary product, and any shape, various dimensions, basis weight, and the like according to the desired leakage preventing performance, breathability, strength, and the like can be employed.

### (Composite Absorbent Body)

In the light incontinence pad 1, the composite absorbent body 4 has a longitudinally elongated outer shape that centers about the central part in the lengthwise direction L and the width direction W of the light incontinence pad 1, extends in a wide region in the lengthwise direction L from the vicinity of one side end edge to the vicinity of the other side end edge in the lengthwise direction L, and also extends in the width direction W in a wide region from the vicinity of the one side end edge to the vicinity of the other side end edge in the width direction W, in which two lengthwise end edges protrude in an arc shape toward the lengthwise outer side in a plan view, as shown in FIG. 1.

More specifically, the composite absorbent body 4 has a constricted portion having a relatively short widthwise length compared with other portions in the lengthwise central part in a plan view, and further has a minimum width portion having the minimum width of the composite absorbent body 4 in the constricted portion, and a maximum width portion having the maximum width of the composite absorbent body 4 on the lengthwise outer side of the constricted portion.

The composite absorbent body 4 is formed of a predetermined water-absorbent member that is arranged between the top sheet 2 and the back sheet 3 in the thickness direction of the light incontinence pad 1 and is capable of absorbing and holding a bodily fluid such as urine that has permeated the top sheet 2 and the absorbent member is formed of a water-absorbent material such as a polymer absorbent, hydrophilic fibers, or superabsorbent polymer, which will be described later, and a sheet such as a tissue that holds the water-absorbent material. That is, the composite absorbent body means a water-absorbent member formed of a water-absorbent material that can absorb and hold a bodily fluid, and a sheet that holds the water-absorbent material.

It should be noted that in the light incontinence pad 1, the composite absorbent body 4 is joined to each of the top sheet 2 and the back sheet 3 with any adhesive such as a hot-melt adhesive.

As described above, the composite absorbent body 4 includes, as essential constituent components, a polymer absorbent having a hydrophilic continuous skeleton and continuous pores and having a unique ion exchange ability, and a superabsorbent polymer as described above. The polymer absorbent will be described later, but the superabsorbent polymer is a powder or granular matter formed of a superabsorbent polymer such as a sodium acrylate copolymer well-known in the art, and is referred to as super absorbent polymer (SAP). It should be noted that the specific type of the superabsorbent polymer (SAP) is not particularly limited, and for example, an acrylic acid-based SAP having cations on the surface thereof can be suitably used. The absorption performance (particularly, the water absorption amount, water retention amount, and absorption rate) of the acrylic acid-based SAP in which cations are present on the surface is particularly easily adversely affected by the ions in the bodily fluid (particularly, water absorption amount, water retention amount, and absorption rate). However, even in a case where the composite absorbent body 4 contains such SAP, when the polymer absorbent absorbs and temporarily holds the bodily fluid, the ions in the bodily fluid can be ion-exchanged with the -COOH group and the -COONa group to modify the bodily fluid. Therefore, variation in the absorption performance of the SAP is less likely to occur, and the absorption performance can be stably exhibited as an absorbent body.

It should be noted that the composite absorbent body 4 may include only the polymer absorbent and the SAP as the water-absorbent material, or may further include a water-absorbent material well-known in the art in addition to these materials. Examples of such a water absorbent material include hydrophilic fibers and the like, and more specifically, cellulose-based fibers such as pulp fibers (for example, pulverized pulp), cotton, rayon, and acetate and the like can be used.

It should be noted that the composite absorbent body 4 may have a configuration in which such a polymer absorbent, SAP, and any water-absorbent material are covered with a wrap sheet such as a tissue having hydrophilicity.

It should be noted that, in the present invention, the outer shape, various dimensions, basis weight, and the like of the composite absorbent body are not particularly limited as long as the effects of the present invention are not impaired, and any outer shape, various dimensions, basis weight, and the like according to the desired water absorption, flexibility, strength, and the like can be adopted.

Hereinafter, the polymer absorbent used in the composite absorbent body of the present invention will be described in more detail.

### [Polymer Absorbent]

In the present invention, the polymer absorbent is not particularly limited as long as the polymer absorbent has a hydrophilic continuous skeleton and continuous pores, contains at least a -COOH group and a -COONa group as ion exchange groups, and has the unique ion exchange ability that the total ion exchange capacity of the -COOH group and the -COONa group per unit mass in a dry state is 4.0 mg equivalent/g or more. As such a polymer absorbent, for example, there is provided a polymer compound that is a hydrolysate of a crosslinked polymer of two or more monomers containing at least a (meth)acrylic acid ester and has at least one or more hydrophilic groups in the functional group. More specifically, there is provided a polymer compound that is a hydrolysate of a crosslinked polymer of a (meth)acrylic acid ester and a compound containing two or more vinyl groups in one molecule and has at least a -COOH group and a -COONa group. Such a polymer absorbent is an organic porous body having at least one or more -COONa groups in one molecule and further has a -COOH group. In the skeleton of the porous body, -COONa groups are substantially uniformly distributed.

When the polymer absorbent is a hydrolysate of a crosslinked polymer of a (meth)acrylic acid ester and a compound containing two or more vinyl groups in one molecule, as will be described later, a hydrophilic continuous skeleton easily extends (that is, easily expands) when a bodily fluid such as urine is absorbed, and the continuous pores are easily widened. Therefore, a larger amount of bodily fluid can be more rapidly taken into the continuous pores. Accordingly, the composite absorbent body including the polymer absorbent can exhibit higher absorption performance as an absorbent body, can steadily modify a larger amount of bodily fluid, and can make it more difficult to cause variation in the absorption performance of the SAP.

It should be noted that in the present specification, the term "(meth)acrylic acid ester" refers to an acrylic acid ester or a methacrylic acid ester.

In the polymer absorbent formed of a hydrolysate of a crosslinked polymer of a (meth)acrylic acid ester and divinylbenzene, a hydrophilic continuous skeleton is formed by an organic polymer having at least a -COONa group and a -COOH group, and has continuous holes (continuous pores) that serve as absorption sites for a liquid to be absorbed (that is, a bodily fluid such as urine) are provided between the skeletons.

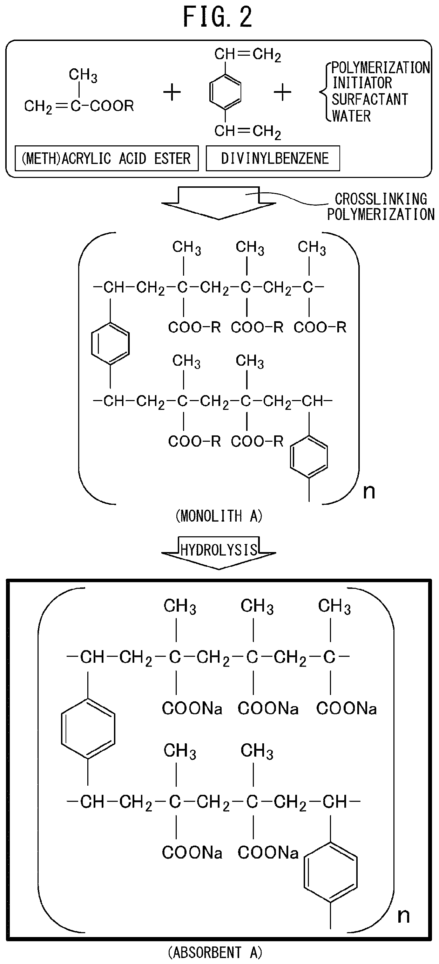

It should be noted that since in the hydrolysis treatment, the -COOR group (that is, the carboxylic acid ester group) of the crosslinked polymer is converted into a -COONa group or a -COOH group (refer to FIG. 2), the polymer absorbent may have a -COOR group.

The presence of the -COOH group and the -COONa group in the organic polymer that forms the hydrophilic continuous skeleton and the total ion exchange capacity of the -COOH group and the -COONa group per unit mass in a dry state can be confirmed by analysis using infrared spectrophotometry and a method of quantifying weakly acidic ion exchange groups.

Here, FIG. 2 is a diagram illustrating a manufacturing process of an absorbent A which is an example of a polymer absorbent. In FIG. 2, the upper figure shows polymer constituent raw materials, the middle figure shows a monolith A as a crosslinked polymer of a (meth)acrylic acid ester and divinylbenzene, and the lower figure shows the absorbent A obtained by subjecting the monolith A in the middle figure to hydrolysis and drying.

Hereinafter, an absorbent A formed of a hydrolysate of a crosslinked polymer of a (meth)acrylic acid ester and divinylbenzene, which is an example of a polymer absorbent, will be described.

It should be noted that the polymer absorbent is not limited to such an absorbent A, and may be a hydrolysate of a crosslinked polymer of a (meth)acrylic acid ester and a compound having two or more vinyl groups in one molecule, or a hydrolysate of a crosslinked polymer of two or more types of monomers containing at least a (meth)acrylic acid ester, or the like.

However, when the polymer absorbent is a monolithic absorbent, the bodily fluid can be rapidly absorbed, and the bodily fluid temporarily held in the polymer absorbent can be more steadily transferred to the SAP. Therefore, the composite absorbent body including the polymer absorbent can stably exhibit further excellent absorption performance.

It should be noted that in the following description, the "monolith A" is an organic porous body formed of a crosslinked polymer of a (meth)acrylic acid ester and divinylbenzene before being subjected to hydrolysis, and will also be referred to as a "monolithic organic porous body".

The "absorbent A" is a hydrolysate of a crosslinked polymer (monolith A) of a (meth)acrylic acid ester and divinylbenzene after being subjected to hydrolysis and drying. It should be noted that the absorbent A is assumed to be in the dry state in the following description.

First, the structure of the absorbent A will be described.

As described above, the absorbent A has a hydrophilic continuous skeleton and continuous pores. The absorbent A, which is an organic polymer having a hydrophilic continuous skeleton, is obtained by performing crosslinking polymerization using a (meth)acrylic acid ester, which is a polymerization monomer, and divinylbenzene, which is a crosslinking monomer, and then hydrolyzing the obtained crosslinked polymer (monolith A) as shown in FIG. 2.

The organic polymer that forms the hydrophilic continuous skeleton has, as constituent units, polymerization residue of an ethylene group (hereinafter, referred to as a "constituent unit X") and a crosslinked polymerization residue of divinylbenzene (hereinafter, referred to as a "constituent unit Y").

Further, the polymerization residue (constituent unit X) of the ethylene group in the organic polymer that forms the hydrophilic continuous skeleton has both the -COOH group and the - COONa group generated by the hydrolysis of the carboxylic acid ester group. It should be noted that, in a case where the polymerization monomer is a (meth)acrylic acid ester, the polymerization residue of the ethylene group (constituent unit X) has a -COONa group, a -COOH group, and an ester group.

In the absorbent A, the ratio of the crosslinked polymerization residue (constituent unit Y) of divinylbenzene in the organic polymer that forms the hydrophilic continuous skeleton is, for example, 0.1 to 30 mol%, and preferably 0.1 to 20 mol%, with respect to all of the constituent units. For example, in the absorbent A containing butyl methacrylate as the polymerization monomer and divinylbenzene as the crosslinking monomer, the ratio of the crosslinked polymerization residue (constituent unit Y) of divinylbenzene in the organic polymer that forms the hydrophilic continuous skeleton is, for example, about 3 mol%, preferably 0.1 to 10 mol%, and more preferably 0.3 to 8 mol%, with respect to all constituent units.

It should be noted that, when the ratio of the crosslinked polymerization residue of divinylbenzene in the organic polymer that forms the hydrophilic continuous skeleton is 0.1 mol% or more, it becomes difficult to decrease the strength of the absorbent A, and, when the ratio of the crosslinked polymerization residue of divinylbenzene is 30 mol% or less, it becomes difficult to decrease the absorption amount of the liquid to be absorbed.

In the absorbent A, the organic polymer that forms the hydrophilic continuous skeleton may be formed of only the constituent unit X and the constituent unit Y, or may be formed of, in addition to the constituent unit X and the constituent unit Y, a constituent unit other than the constituent unit X and the constituent unit Y, such as a polymerization residue of monomers other than (meth)acrylic acid ester and divinylbenzene.

As the constituent unit other than the constituent unit X and the constituent unit Y, for example, there are provided polymerization residues of monomers such as styrene, α-methylstyrene, vinyltoluene, vinylbenzyl chloride, glycidyl (meth)acrylate, isobutene, butadiene, isoprene, chloroprene, vinyl chloride, vinyl bromide, vinylidene chloride, tetrafluoroethylene, (meth)acrylonitrile, vinyl acetate, ethylene glycol di(meth)acrylate, tripropylene glycol di(meth)acrylate, trimethylol propane tri(meth)acrylate and the like.

It should be noted that the ratio of the constituent unit other than the constituent unit X and the constituent unit Y in the organic polymer that forms the hydrophilic continuous skeleton is, for example, 0 to 50 mol%, and preferably 0 to 30 mol%, with respect to all of the constituent units.

Further, it is preferable that the thickness of the hydrophilic continuous skeleton of the absorbent A is 0.1 to 100 µm. When the thickness of the hydrophilic continuous skeleton of the absorbent A is 0.1 µm or more, spaces (pores) for taking in a liquid (bodily fluid) to be absorbed in the porous body is less likely to collapse during absorption, and the absorption amount is less likely to decrease. On the other hand, when the thickness of the hydrophilic continuous skeleton is 100 µm or less, an excellent absorption rate can be easily obtained.

It should be noted that since the pore structure of the hydrophilic continuous skeleton of the absorbent A is an open cell structure, the thickness of the continuous skeleton is measured at the thickness evaluation point, which is the cross section of the skeleton appearing in a test piece for electron microscope measurement. The continuous skeleton is formed by spaces between water droplets removed by dehydration and drying after hydrolysis described later, and therefore the skeleton is often polygonal. Therefore, the thickness of the continuous skeleton is defined as the average value of the diameter (µm) of a circle that circumscribes the polygonal cross section. In addition, in rare cases, a small hole may exist in the polygon, but in such cases, the circumscribed circle of the polygonal cross section that surrounds the small hole is measured.

Further, it is preferable that the average diameter of the continuous pores of the absorbent A is 1 µm to 1000 µm. When the average diameter of the continuous pores of the absorbent A is 1 µm or more, the spaces (pores) for taking in the liquid (bodily fluid) to be absorbed in the porous body become less likely to collapse during absorption, and the absorption rate is less likely to decrease. On the other hand, when the average diameter of the continuous pores is 1000 µm or less, an excellent absorption rate can be easily obtained. Therefore, the composite absorbent body including such an absorbent A can stably exhibit excellent absorption performance.

Particularly, in a case where the porosity per unit volume of the polymer absorbent described later is 85% or more and the average diameter of the continuous pores is 1 µm to 1000 µm, the bodily fluid can be absorbed with a larger number of pores and modified, and thus there is an advantage that further excellent ion exchange efficiency can be embodied.

It should be noted that the average diameter (µm) of the continuous pores of the absorbent A can be measured by mercury intrusion porosimetry, and the maximum value of the pore distribution curve obtained by such a mercury intrusion porosimetry is employed. For a sample used for measurement of the average diameter of the continuous pores, regardless of the ionic form of the absorbent A, a sample dried in a vacuum dryer set at a temperature of 50°C for 18 hours or longer is used. It should be noted that the final reaching pressure is set to 0 Torr.

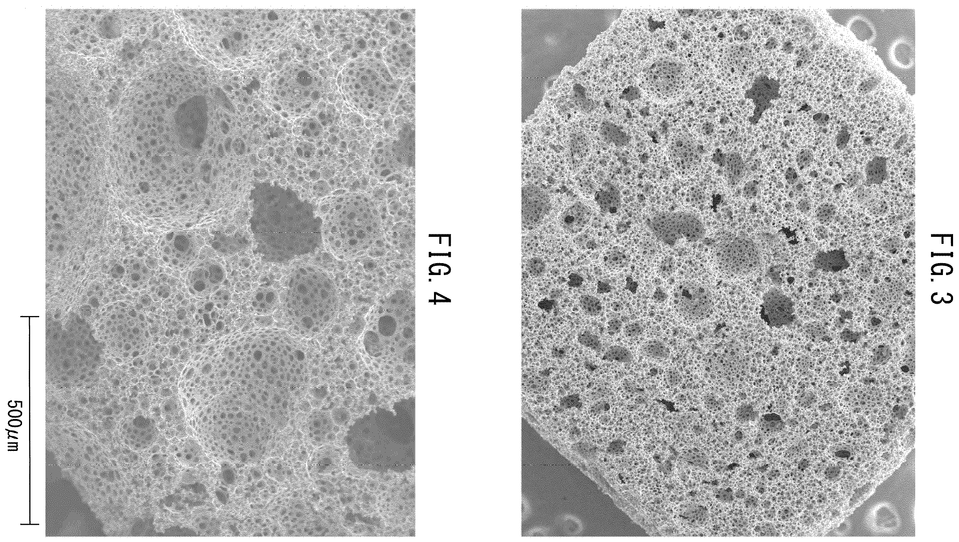

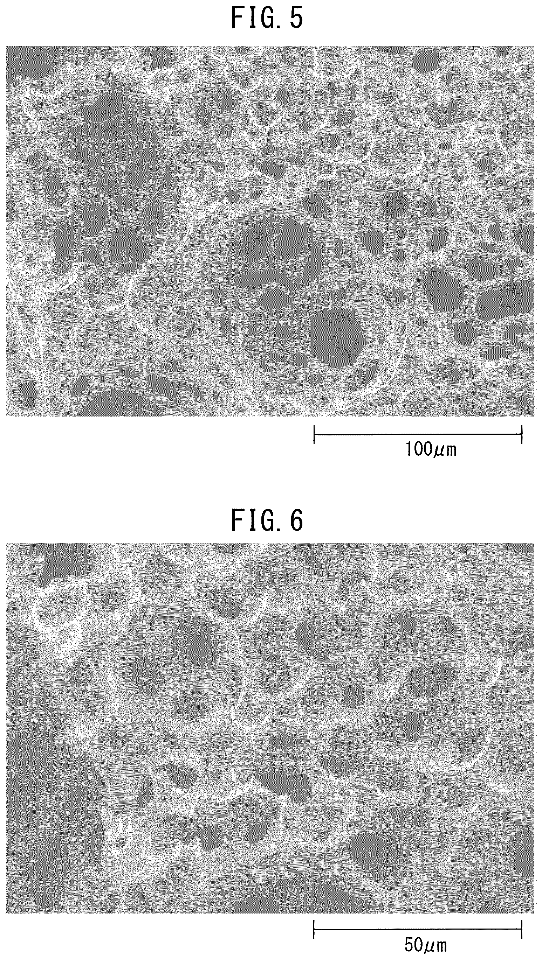

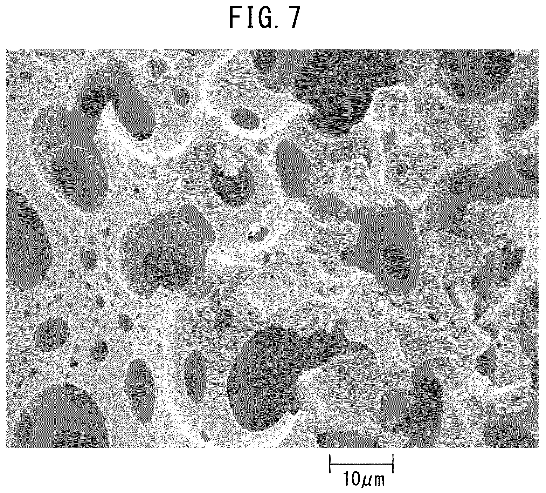

Here, FIG. 3 is an SEM photograph of the absorbent A at a magnification of 50 times, FIG. 4 is an SEM photograph of the absorbent A at a magnification of 100 times, FIG. 5 is an SEM photograph of the absorbent A at a magnification of 500 times, FIG. 6 is an SEM photograph of the absorbent A at a magnification of 1000 times, and FIG. 7 is an SEM photograph of the absorbent A at a magnification of 1500 times.

The absorbent A shown in FIGS. 3 to 7 is an example of an absorbent containing butyl methacrylate as the polymerization monomer and divinylbenzene as the crosslinking monomer, and has a cubic structure having 2 mm sides.

The absorbent A shown in FIGS. 3 to 7 has a large number of bubble-shaped macropores, and further has a portion in which these bubble-shaped macropores overlap each other. The absorbent A has an open cell structure in which portion in which these macropores overlap each other forms a common opening (mesopore), that is, an open cell structure (continuous macropore structure).

The portion in which the macropores overlap each other forms a common opening (mesopore) having an average diameter of 1 to 1000 µm in a dry state, preferably 10 to 200 µm, and particularly preferably 20 to 100 µm, and most of the common openings (mesopores) have an open pore structure. When the average diameter of the mesopores in the dry state is 1 µm or more, the absorption rate of the liquid to be absorbed becomes more favorable. On the other hand, when the average diameter of the mesopores in the dry state is 1000 µm or less, the absorbent A is less likely to be embrittled.

It should be noted that such an overlap between the macropores is approximately 1 to 12 pieces in one macropore, and is approximately 3 to 10 pieces in many cases.

Further, the absorbent A having such an open cell structure makes it possible to uniformly form a macropore group and a mesopore group, and also has the advantage of making it possible to remarkably increase the pore volume and specific surface area compared with a particle agglomerated porous body such as that described in Japanese Patent Application Publication No. H08-252579.

It should be noted that the total pore volume of the pores of the absorbent A is preferably 0.5 to 50 mL/g, and more preferably 2 to 30 mL/g. When the total pore volume of the absorbent A is 0.5 mL/g or more, spaces (pores) for taking in the liquid (bodily fluid) to be absorbed in the porous body become less likely to collapse during absorption, and the absorption amount and the absorption rate are less likely to decrease. On the other hand, when the total pore volume of the absorbent A is 50 mL/g or less, the strength of the absorbent A is less likely to decrease.

It should be noted that the total pore volume can be measured by the mercury intrusion porosimetry. As a sample for measurement of the total pore volume, regardless of the ionic form of the absorbent A, a sample obtained by drying in a vacuum dryer set at a temperature of 50°C for 18 hours or longer is used. It should be noted that the final reaching pressure is set to 0 Torr.

Hereinafter, the appearance in a case where the absorbent A and a liquid such as a bodily fluid (hereinafter, simply referred to as a "bodily fluid") come into contact with each other will be described. However, the same applies to a case where the composite absorbent body 4 containing the absorbent A comes into contact with the bodily fluid.

As shown in FIGS. 3 to 7, the continuous pores of the absorbent A are pores in which a plurality of pores are continuous with each other, and the fact that a large number of pores are located can be visually recognized from the external appearance as well. When the bodily fluid such as urine comes into contact with the absorbent A having a large number of pores, the hydrophilic continuous skeleton first instantaneously takes in a part of the bodily fluid by osmotic pressure and extends (that is, expands). The continuous skeleton extends in substantially all directions. In the absorbent A, as the outer shape of the absorbent A increases due to the extension of the continuous skeleton during water absorption, the size of the pores also increases. When the size of the pores is increased in this manner, the volume in the pore is increased, and therefore, the amount of bodily fluid that can be stored in the pores is also increased. The absorbent A which is increased due to the absorption of a predetermined amount of bodily fluid in this manner is capable of further absorbing a predetermined amount of bodily fluid into the expanded pores due to the capillary phenomenon.

It should be noted that, while the bodily fluid absorbed into the hydrophilic continuous skeleton of the absorbent A is less likely to be released from the continuous skeleton (that is, less likely to be separated), the bodily fluid absorbed into the continuous pores is likely to be separated. Therefore, in the composite absorbent body, the bodily fluid absorbed into the continuous pores is separated, transferred to the SAP having a high water retention capacity, and steadily held in the SAP.

Further, in the bodily fluid absorbed by the absorbent A, the amount of bodily fluid that remains in the pores is larger than the amount of bodily fluid absorbed in the hydrophilic continuous skeleton. Since most of the bodily fluid absorbed in the absorbent A is absorbed by storing the bodily fluid in the pores due to the capillary phenomenon, the higher the porosity (that is, the void volume of the pores per unit volume of the absorbent A), which is the ratio of the void volume of the pores (total pore volume), the more the bodily fluid can be absorbed.

The porosity per unit volume of the polymer absorbent is preferably 85% or more, and more preferably 90% or more. When the porosity per unit volume of the polymer absorbent is 85% or more, a larger amount of bodily fluid can be absorbed and steadily modified, and thus variation in the absorption performance of the SAP can be made much less likely to occur. Accordingly, the composite absorbent body containing such a polymer absorbent can more stably and satisfactorily exhibit the absorption performance as an absorbent body.

For example, when the porosity of the absorbent A shown in FIGS. 3 to 7 described above is calculated, the following is obtained.

First, the specific surface area of the absorbent A obtained by the mercury intrusion porosimetry is 400 m²/g, and the pore volume is 15.5 mL/g. This pore volume of 15.5 mL/g means that the volume of the pores in 1 g of the absorbent A is 15.5 mL.

Here, in a case of assuming that the specific gravity of the absorbent A is 1 g/mL, the volume of the pores occupied in 1 g of the absorbent A, that is, the pore volume, is 15.5 mL, and the volume of 1 g of the absorbent A is 1 mL.

Then, the total volume (volume) of 1 g of the absorbent A becomes 15.5 + 1 (mL), and the ratio of the pore volume in the total volume becomes the porosity. Therefore, the porosity of the absorbent A becomes 15.5/(15.5 + 1) x 100 = 94%.

The absorbent A having such a hydrophilic continuous skeleton and continuous pores, that is, the polymer absorbent is applied to a composite absorbent body for absorbing a bodily fluid such as urine, such as the composite absorbent body 4 of the light incontinence pad 1 described above, in the form of, for example, a particle, a sheet, or the like.

Then, since the polymer absorbent contains at least a -COOH group and a -COONa group as ion exchange groups, and has the unique ion exchange ability that the total ion exchange capacity of the -COOH group and the -COONa group per unit mass in a dry state is 4.0 mg equivalent/g or more as described above, when the polymer absorbent absorbs and temporarily holds the bodily fluid, the ions (particularly, divalent ions such as Ca²⁺ and Mg²⁺) in the bodily fluid can be ion-exchanged with the -COOH group and the -COONa group to modify the bodily fluid into a bodily fluid that is less likely to have an adverse effect on the absorption performance (particularly, water absorption amount, water retention amount, and absorption rate) of the superabsorbent polymer (SAP). Accordingly, since the composite absorbent body to which such a polymer absorbent is applied can transfer the bodily fluid to the SAP after modifying the bodily fluid with the polymer absorbent, variation in the absorption performance of the SAP is less likely to occur, and the absorption performance can be stably exhibited as an absorbent body.

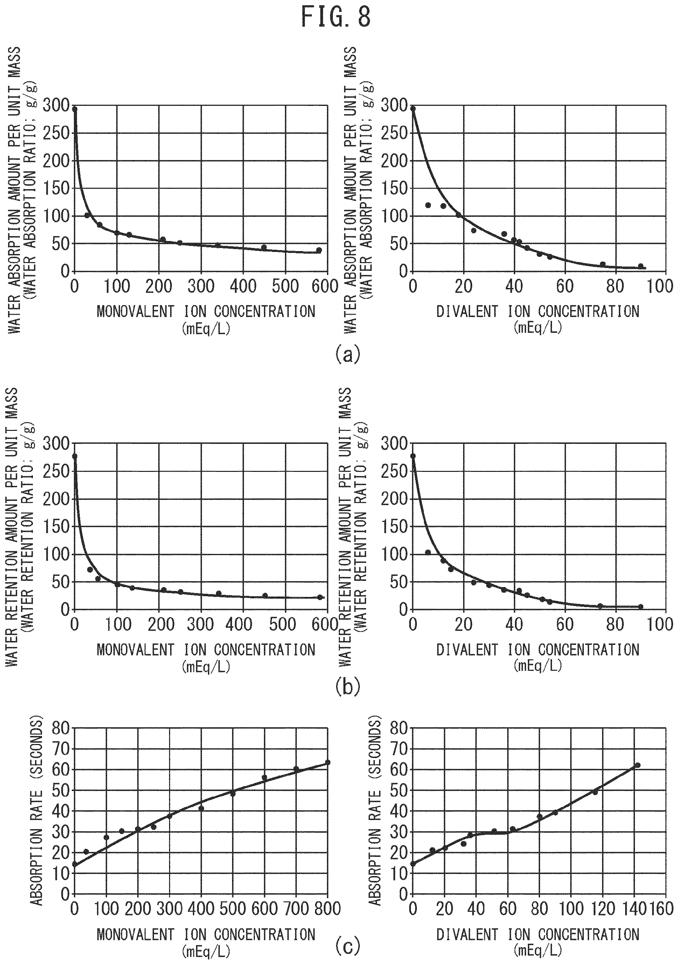

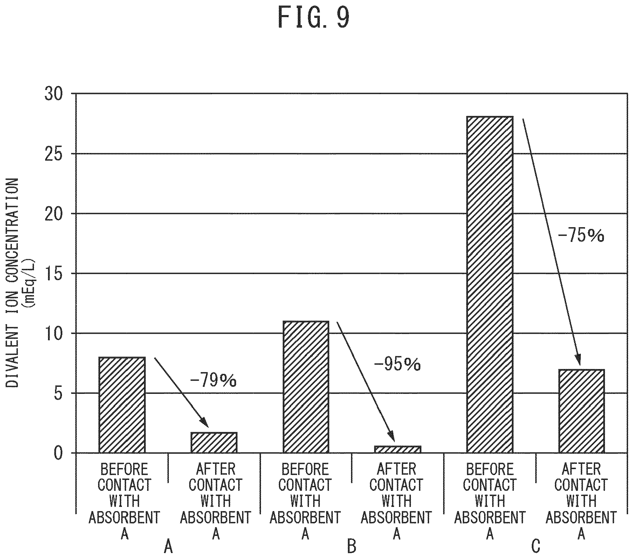

Here, FIG. 8 is a graph showing the relationship between the monovalent and divalent ion concentration in the bodily fluid (urine) and the absorption performance (water absorption amount, water retention amount, and absorption rate) of the SAP, and FIG. 9 is a graph showing the influence of the absorbent A, which is an example of a polymer absorbent, on the divalent ion concentration in the bodily fluid (urine).

As shown in FIG. 8, it can be seen that all of the water absorption amount, the water retention amount, and the absorption rate of the SAP decrease as the monovalent and divalent ion concentration in the bodily fluid (urine) increases. Particularly, it can be seen that the divalent ion concentration has a large influence on the absorption performance of the SAP, and even in a very small amount, the water absorption amount, the water retention amount, and the absorption rate of the SAP greatly decrease.

As described above, the ion concentration in the bodily fluid (urine) has a large adverse effect on the absorption performance of the SAP. However, since variation occurs in the ion concentration for each bodily fluid (that is, the type and individual difference of bodily fluid) due to differences in dietary habits (for example, ingredients of taken food and drink and the like) and living environments (for example, frequency of sweating and urination and the like), variation also occurs in the absorption performance of the SAP according to the variation.

However, since the absorbent A, which is an example of the present invention, contains at least a -COOH group and a -COONa group as ion exchange groups, and has the unique ion exchange ability that the total ion exchange capacity of the -COOH group and the -COONa group per unit mass in a dry state is 4.0 mg equivalent/g or more, as shown in FIG. 9, the ions (particularly, divalent ions) in the bodily fluid (urine) can be ion-exchanged with the -COOH group and the - COONa group and the ion concentration in the bodily fluid (urine) can be significantly reduced. That is, the bodily fluid (urine) can be modified into a bodily fluid that is less likely to have an adverse effect on the absorption performance of the SAP.

It should be noted that, in the graph shown in FIG. 9, the change rate of the ion concentration before and after the contact between the actual urine (actual urines A, B, and C) of three humans having different divalent ion concentrations and the absorbent A (that is, the ion exchange rate of the absorbent A) is measured as follows.

First, the divalent ion concentration (mEq/L) of each of the three actual urines A, B, and C are measured using an ion meter (HORIBA Compact Calcium Ion Meter LAQUAtwin-Ca-11, manufactured by HORIBA Advanced Techno, Co., Ltd.). The measured ion concentration is defined as the ion concentration "before the contact with the absorbent A". Next, 0.2 g of the polymer absorbent (absorbent A) is placed in a glass filter (climbing glass filter, model number: 0777-01-101, outer diameter x foot length (mm): ϕ7 x 80, filter diameter: ϕ20 mm, capacity: 30 mL, material: borosilicate glass, pore diameter: 100 to 120 µm), 30 mL of the actual urine is poured, and the divalent ion concentration (mEq/L) of the obtained filtrate is measured using the above-mentioned ion meter. The measured ion concentration is defined as the ion concentration of "after the contact with the absorbent A".

The amount (mEq/L) of the change in the ion concentration before and after the contact with the absorbent A is calculated by subtracting the ion concentration after the contact with the absorbent A from the ion concentration before the contact with the absorbent A. Further, the amount (mEq/L) of the change in the ion concentration before and after the contact with the absorbent A is divided by the ion concentration before the contact with the absorbent A and multiplied by 100, thereby calculating the change rate (%) of the divalent ion concentration in each of the actual urines A, B, and C.

It should be noted that all of the above measurements are performed under the conditions of temperature of 25°C and a humidity of 60%.

As described above, the absorbent A of the present invention has the unique ion exchange ability that is not found in conventional water-absorbent materials, can rapidly absorb and temporarily hold the bodily fluid such as urine, and can ion-exchange the ions (particularly, divalent ions) in the bodily fluid to modify the bodily fluid into a bodily fluid that is less likely to have an adverse effect on the absorption performance of the SAP.

Accordingly, since the composite absorbent body containing such an absorbent A (polymer absorbent) can transfer the bodily fluid to the SAP after modifying the bodily fluid, variation in the absorption performance of the SAP is less likely to occur, and the absorption performance can be stably exhibited as an absorbent body.

It should be noted that, in the present invention, the total ion exchange capacity of the -COOH group and the -COONa group per unit mass of the polymer absorbent in a dry state is preferably 6.0 mg equivalent/g or more, and more preferably 8.0 mg equivalent/g or more.

It should be noted that, in the present invention, it is preferable that the ion exchange rate of the polyvalent ions (that is, ions having a valence of 2 or more) of the polymer absorbent is 50% or more. When the ion exchange rate of the polyvalent ions of the polymer absorbent is 50% or more, the bodily fluid can be more reliably modified, and variation in the absorption performance of the SAP can be made much less likely to occur. Therefore, the composite absorbent body containing such a polymer absorbent can more stably exhibit absorption performance as an absorbent body. It should be noted that the ion exchange rate of the polyvalent ions of the polymer absorbent is more preferably 60% or more, and still more preferably 70% or more.

Here, the ion exchange rate of the polyvalent ions of the polymer absorbent can be measured by any measurement method such as ICP emission spectrometry, IC analysis method, or an atomic absorption spectrometry method. For example, the ion exchange rate of the divalent ions can be measured as follows.

### <Method for Measuring Ion Exchange Rate of Divalent Ions of Polymer Absorbent>

(1) Artificial urine is prepared by dissolving 200 g of urea, 80 g of sodium chloride, 8 g of magnesium sulfate, 3 g of calcium chloride, and approximately 1 g of a dye (blue No. 1) in 10 L of ion exchange water.
(2) The divalent ion concentration (mEq/L) of the prepared artificial urine is measured using an ion meter (HORIBA Compact Calcium Ion Meter LAQUAtwin-Ca-11, manufactured by Horiba Advanced Techno Co., Ltd.). The measured ion concentration is defined as "the ion concentration before the contact with the polymer absorbent".
(3) 0.2 g of the polymer absorbent which is a sample for measurement is placed in a glass filter (climbing glass filter, model number: 0777-01-101, outer diameter x foot length (mm): ϕ7 x 80, filter diameter: ϕ20 mm, capacity: 30 mL, material: borosilicate glass, pore diameter: 100 to 120 µm), 30 mL of the artificial urine is poured, and the divalent ion concentration (mEq/L) of the obtained filtrate is measured using the above-mentioned ion meter. The measured ion concentration is defined as "the ion concentration after the contact with the polymer absorbent".
(4) The amount (mEq/L) of the change in the ion concentration before and after the contact with the polymer absorbent is calculated by subtracting the ion concentration after the contact with the polymer absorbent from the ion concentration before the contact with the polymer absorbent. Further, the amount (mEq/L) of the change in the ion concentration before and after the contact with the polymer absorbent is divided by the ion concentration before the contact with the polymer absorbent and multiplied by 100, thereby calculating the change rate (%) of the divalent ion concentration. In the present specification, this "change rate (%) of the divalent ion concentration" is defined as "the divalent ion exchange rate of the polymer absorbent".

It should be noted that all of the above measurements are performed under the conditions of temperature of 25°C and a humidity of 60%.

Further, in a case where the sample for measurement (polymer absorbent) is collected from a product of a sanitary product and used, the sample for measurement (polymer absorbent) can be obtained according to the following <Method for Collecting Sample for Measurement (Polymer Absorbent)> .

### <Method for Collecting Sample for Measurement (Polymer Absorbent)>

(1) The top sheet or the like is peeled off from the product of the sanitary product to expose the absorbent body.
(2) The object to be measured (polymer absorbent) is dropped from the exposed absorbent body, and a substance other than the object to be measured (in a particulate state) (for example, pulp, synthetic resin fibers, or the like) is removed with tweezers or the like.
(3) A microscope or a simple loupe is used as magnifying observation means, and the object to be measured is collected using the tweezers or the like, while observing at a magnification capable of recognizing the difference from SAP or at a magnification capable of visually recognizing the pores of the porous body. It should be noted that the magnification of the simple loupe is not particularly limited as long as the pores of the porous body can be visually recognized, and, for example, a magnification of 25 to 50 times can be given.
(4) The object to be measured collected in this manner is used as the sample for measurement in various measurement methods.

Further, in the present invention, it is preferable that a water absorption amount per unit mass of the polymer absorbent is 30 g/g or more. When the polymer absorbent has a certain water absorption amount or more as described above, a larger amount of bodily fluid can be absorbed and steadily modified. Therefore, variation in the absorption performance of the SAP can be made much less likely to occur. Therefore, the composite absorbent body containing such a polymer absorbent can more stably exhibit absorption performance as an absorbent body.

It should be noted that the water absorption amount per unit mass of the polymer absorbent is more preferably 40 g/g or more, and still more preferably 50 g/g or more.

Here, the water absorption amount of the polymer absorbent per unit mass can be measured as follows.

### <Method for Measuring Water Absorption Amount Per Unit Mass of Polymer Absorbent>

(1) 1 g of a sample for measurement (polymer absorbent) is sealed in a mesh bag (N-NO255HD 115 (standard width: 115 cm, 255 mesh/2.54 cm, opening: 57 µm, wire diameter: 43 µm, thickness: 75 µm), manufactured by NBC Meshtec Inc.) which is cut into 10 cm squares. It should be noted that the mass (g) of the mesh bag is measured in advance. Further, in a case where the sample for measurement (polymer absorbent) is collected from a product of a sanitary product and used, the sample for measurement (polymer absorbent) can be obtained according to the above <Method for Collecting Sample for Measurement (Polymer Absorbent)>.
(2) The mesh bag containing the sample sealed therein is immersed in physiological saline (0.9% sodium chloride aqueous solution) for 1 hour.
(3) The mass (g) after the mesh bag is suspended for 5 minutes and drained is measured.
(4) The water absorption amount (g) of the sample is calculated by subtracting the mass (= 1 g) of the sample and the total mass of the mesh bag from the mass of the mesh bag after draining measured in (3) above, and the water absorption amount is further divided by the mass (= 1 g) of the sample to obtain the water absorption amount (g/g) per unit mass of the sample (polymer absorbent).

It should be noted that all of the above measurement methods are performed under conditions of a temperature of 25°C and a humidity of 60%.

Hereinafter, a method for manufacturing such a polymer absorbent will be described in detail using the above-described absorbent A as an example.

### [Method for Manufacturing Polymer Absorbent]

As shown in FIG. 2, the above-described absorbent A can be obtained by allowing the absorbent A to pass through a crosslinking polymerization step and a hydrolysis step. Hereinafter, each of these steps will be described.

### (Crosslinking Polymerization Step)

First, an oil-soluble monomer for crosslinking polymerization, a crosslinking monomer, a surfactant, water, and optionally a polymerization initiator are mixed to obtain a water-in-oil emulsion. This water-in-oil emulsion is an emulsion in which water droplets are dispersed in an oil phase that serves as a continuous phase.

As shown in the upper figure of FIG. 2, in the above-described absorbent A, a monolith A is obtained by performing crosslinking polymerization using butyl methacrylate, which is (meth)acrylic acid ester, as an oil-soluble monomer, divinylbenzene as a crosslinking monomer, sorbitan monooleate as a surfactant, and further isobutyronitrile as a polymerization initiator.

Specifically, in the absorbent A, as shown in the upper figure of FIG.2, first, 9.2 g of t-butyl methacrylate as an oil-soluble monomer, 0.28 g of divinylbenzene as a crosslinking monomer, 1.0 g of sorbitan monooleate (hereinafter, abbreviated as "SMO") as a surfactant, and 0.4 g of 2,2'-azobis(isobutyronitrile) as a polymerization initiator are mixed and uniformly dissolved.

Next, the mixture of t-butyl methacrylate, divinylbenzene, SMO, and 2,2'-azobis(isobutyronitrile) is added to 180 g of pure water, and a vacuum stirring defoaming mixer (manufactured by EME. Corp.), which is a planetary motion type stirring device, is used to perform stirring under reduced pressure to obtain a water-in-oil emulsion.

Further, the emulsion is rapidly transferred to a reaction vessel, sealed, and left to stand still and polymerized at 60°C for 24 hours. After completion of the polymerization, the content is taken out, extracted with methanol, and dried under reduced pressure to obtain a monolith A having a continuous macropore structure. It should be noted that, as a result of observing the internal structure of the monolith A by SEM, the monolith A had an open cell structure and the thickness of the continuous skeleton is 5.4 µm. Further, the average diameter of the continuous pores measured by the mercury intrusion porosimetry is 36.2 µm, and the total pore volume is 15.5 mL/g.

It should be noted that the content of divinylbenzene with respect to all the monomers is preferably 0.3 to 10 mol%, and more preferably 0.3 to 5 mol%. Further, the ratio of divinylbenzene to the total of butyl methacrylate and divinylbenzene is preferably 0.1 to 10 mol%, and more preferably 0.3 to 8 mol%. It should be noted that in the above-described absorbent A, the ratio of butyl methacrylate with respect to the total of butyl methacrylate and divinylbenzene is 97.0 mol% and the ratio of divinylbenzene with respect to the total of butyl methacrylate and divinylbenzene is 3.0 mol%.

The amount of the surfactant added can be set depending on the type of the oil-soluble monomer and the size of the desired emulsion particle (macropore), and it is preferable that the amount of the surfactant is set to be in a range of about 2 to 70% with respect to the total amount of the oil-soluble monomer and the surfactant.

It should be noted that, in order to control the bubble shape and the size of the monolith A, alcohols such as methanol and stearyl alcohol; carboxylic acids such as stearic acid; hydrocarbons such as octane, dodecane, and toluene; cyclic ethers such as tetrahydrofuran and dioxane, and the like may coexist in the polymerization system.

Further, the mixing method for forming the water-in-oil emulsion is not particularly limited, and for example, any mixing method such as a method of mixing each component all together at once, and a method in which oil-soluble components, which are an oil-soluble monomer, a surfactant, and an oil-soluble polymerization initiator, and water-soluble components, which are water or a water-soluble polymerization initiator, are uniformly dissolved separately, and then oil-soluble components and water-soluble components are mixed can be adopted.

Further, the mixing device for forming the emulsion is not particularly limited, and any device such as an ordinary mixer, homogenizer, or high pressure homogenizer can be used according to the desired emulsion particle diameter. Further, a so-called planetary type stirring device in which an object to be treated is placed in a mixing container, and the object to be treated is stirred and mixed by rotating the mixing container in a tilted state while revolving around the revolution axis can be used.

Further, the mixing conditions are not particularly limited, and any stirring rotation rate, any stirring time, and the like can be set depending on the desired emulsion particle diameter. It should be noted that, in the above-described planetary type stirring device, water droplets can be uniformly generated in the W/O emulsion, and any average diameter can be set within a wide range.

As the polymerization conditions of the water-in-oil emulsion, various conditions can be adopted depending on the type of monomer or initiator and the like. For example, in a case of using azobisisobutyronitrile, benzoyl peroxide, potassium persulfate, or the like as the polymerization initiator, polymerization may be conducted by heating at a temperature of 30°C to 100°C for 1 to 48 hours in a sealed container under an inert atmosphere. In a case of using hydrogen peroxide-ferrous chloride, sodium persulfate-sodium acid sulfite, or the like as the polymerization initiator, polymerization may be conducted at a temperature of 0 to 30°C for 1 to 48 hours in a sealed container under an inert atmosphere.

It should be noted that, after the polymerization, the content is taken out and subjected to Soxhlet extraction with a solvent such as isopropanol to remove the unreacted monomer and the residual surfactant, and thus the monolith A shown in the middle figure of FIG. 2 is obtained.

### (Hydrolysis Step)

Subsequently, a step (hydrolysis step) of hydrolyzing the monolith A (crosslinked polymer) to obtain the absorbent A will be described.

First, the monolith A is immersed in dichloroethane to which zinc bromide is added, stirred at 40°C for 24 hours, then brought into contact with methanol, 4% hydrochloric acid, a 4% aqueous solution of sodium hydroxide, and water in this order for hydrolysis, and then dried. Thus, a block-shaped absorbent A is obtained. Further, the block-shaped absorbent A is pulverized to a predetermined size to obtain a particulate absorbent A. It should be noted that the form of the absorbent A is not limited to a particulate form, and, for example, the absorbent A may be shaped into a sheet shape while or after drying.

Further, the method for hydrolyzing the monolith A is not particularly limited, and various methods can be adopted. For example, there is a method of bringing a solvent into contact with a strong base such as sodium hydroxide, or a method of bringing a solvent into contact with a hydrohalic acid such as hydrochloric acid, a Brönsted acid such as sulfuric acid, nitric acid, trifluoroacetic acid, methanesulfonic acid, or p-toluenesulfonic acid, or a Lewis acid such as zinc bromide, aluminum chloride, aluminum bromide, titanium (IV) chloride, cerium chloride/sodium iodide, or magnesium iodide using any of various solvent including aromatic solvents such as toluene and xylene, halogen-based solvents such as chloroform and dichloroethane, ether-based solvents such as tetrahydrofuran and isopropyl ether, amide-based solvents such as dimethylformamide and dimethylacetamide, alcohol-based solvents such as methanol and ethanol, carboxylic acid-based solvents such as acetic acid and propionic acid, or water as the solvent.

Further, among the polymerization raw materials of the organic polymer that forms the hydrophilic continuous skeleton of the absorbent A, the (meth)acrylic acid ester is not particularly limited, C1 to C10 (that is, containing 1 to 10 carbon atoms) alkyl ester of (meth)acrylic acid is preferable, and C4 (that is, containing 4 carbon atoms) alkyl ester of (meth)acrylic acid is particularly preferable.

It should be noted that as a C4 alkyl ester of (meth)acrylic acid, there are provided (meth)acrylic acid t-butyl ester, (meth)acrylic acid n-butyl ester, and (meth)acrylic acid iso-butyl ester.

Further, the monomer used for the crosslinking polymerization may be only (meth)acrylic acid ester and divinylbenzene, or may include (meth)acrylic acid ester and divinylbenzene, and additionally, a monomer other than (meth)acrylic acid ester and divinylbenzene.

In the latter case, the monomer other than (meth)acrylic acid ester and divinylbenzene styrene is not particularly limited, and for example, there are provided styrene, α-methylstyrene, vinyltoluene, vinylbenzyl chloride, glycidyl (meth)acrylate, 2-ethylhexyl (meth)acrylate, isobutene, butadiene, isoprene, chloroprene, vinyl chloride, vinyl bromide, vinylidene chloride, tetrafluoroethylene, (meth)acrylonitrile, vinyl acetate, ethylene glycol di(meth)acrylate, trimethylol propane tri(meth)acrylate, and the like.

It should be noted that, the ratio of the monomer other than (meth)acrylic acid ester and divinylbenzene in all monomers used for the crosslinking polymerization is preferably 0 to 80 mol%, and more preferably 0 to 50 mol%.

Further, the surfactant is not limited to the above-described sorbitan monooleate, and any surfactant that can form a water-in-oil (W/O) emulsion when a monomer for crosslinking polymerization and water are mixed can be used. As such a surfactant, for example, there are provided nonionic surfactants such as sorbitan monolaurate, sorbitan monopalmitate, sorbitan monostearate, sorbitan trioleate, polyoxyethylene group nonylphenyl ether, polyoxyethylene group stearyl ether, and polyoxyethylene group sorbitan monooleate, anionic surfactants such as potassium oleate, sodium dodecylbenzene sulfonate, and dioctyl sodium sulfosuccinate, cationic surfactants such as distearyl dimethyl ammonium chloride, and amphoretic surfactants such as lauryl dimethyl betaine. These surfactants may be used alone or in combination of two or more types.

In addition, a compound that generates radicals by heat and light irradiation is suitably used as the polymerization initiator. The polymerization initiator may be water-soluble or oil-soluble. For example, there are provided azobis (4-methoxy-2,4-dimethylvaleronitrile), azobisisobutyronitrile, azobisdimethylvaleronitrile, azobiscyclohexanenitrile, azobiscyclohexanecarbonitrile, azobis (2-methylpropionamidine) dihydrochloride, benzoyl peroxide, potassium persulfate, ammonium persulfate, hydrogen peroxide-ferrous chloride, sodium persulfate-sodium acid sulfite, and tetramethylthiuram disulfide. However, in some cases, there is a system in which polymerization proceeds with only heating or only light irradiation even when the polymerization initiator is not added. Therefore, in such a system, the addition of the polymerization initiator is not necessary.

It should be noted that, in addition to the light incontinence pad of the above-described embodiment, the composite absorbent body of the present invention can be applied to various sanitary products such as underpants-shaped disposable diapers, tape-type disposable diapers, sanitary napkins, absorbent panty liners, absorbent pads (for example, bedsore pads, puerperal pads and the like), absorbent sheets, breast pads, disposable diapers for pets, absorbent pads for pets, excrement disposal sheets for pets, wet sheets, wet tissues, cosmetic wiping sheets, masks, and the like. Therefore, the bodily fluid that is the liquid to be absorbed of the composite absorbent body is a liquid discharged from a wearer of a sanitary product, and for example, there are provided urine, sweat, feces, menstrual blood, vaginal discharge, breast milk, blood, exudate, and the like.

In addition, the present invention is not limited to the above-described embodiments and the like, and appropriate combinations, substitutions, modifications, and the like can be made without departing from the aspect, gist, and the like of the present invention.

### REFERENCE SIGNS LIST

1: Light incontinence pad
2: top sheet
3: back sheet
4: composite absorbent body

## Claims

1. A composite absorbent body for absorbing a bodily fluid, the composite absorbent body comprising:
a polymer absorbent having a hydrophilic continuous skeleton and a continuous pore; and
a superabsorbent polymer, wherein
the polymer absorbent contains at least a -COOH group and a -COONa group as ion exchange groups, and
a total ion exchange capacity of the -COOH group and the -COONa group per unit mass in a dry state is 4.0 mg equivalent/g or more.

2. The composite absorbent body according to Claim 1, wherein
an ion exchange rate of a polyvalent ion of the polymer absorbent is 50% or more.

3. The composite absorbent body according to Claim 1 or 2, wherein
a water absorption amount per unit mass of the polymer absorbent is 30 g/g or more.

4. The composite absorbent body according to any one of Claims 1 to 3, wherein
in the polymer absorbent, a porosity per unit volume of the polymer absorbent is 85% or more.

5. The composite absorbent body according to any one of Claims 1 to 4, wherein
an average diameter of the continuous pore of the polymer absorbent is 1 µm to 1000 µm.

6. The composite absorbent body according to any one of Claims 1 to 5, wherein
the polymer absorbent is a monolithic absorbent.

7. The composite absorbent body according to any one of Claims 1 to 6, wherein
the polymer absorbent is a hydrolysate of a crosslinked polymer of a (meth)acrylic acid ester and a compound containing two or more vinyl groups in one molecule.

8. The composite absorbent body according to any one of Claims 1 to 7, wherein
the superabsorbent polymer is an acrylic acid-based superabsorbent polymer having a cation present on a surface.

9. A sanitary product comprising:
the composite absorbent body according to any one of Claims 1 to 8.
